# EUROPEAN PATENT APPLICATION

(11) **EP 4 327 861 A1**
(43) Date of publication of application: **28.02.2024**
(21) Application number: 22315189.5
(22) Date of filing: 23.08.2022
(51) Int. Cl.: A61N 1/39

(54) **RESUSCITATION DEVICE**

(71) Applicant: Schiller Medical, 67160 Wissembourg (FR)
(72) Inventor: Jean-Philippe Didon, 67250 Merkwiller-Pechelbronn (FR); Sarah Menetre, 67360 Dieffenbach-les-Woerth (FR); Simon Paulus, 67100 Strasbourg (FR)
(74) Representative: Hepp Wenger Ryffel AG

(57) **Abstract**

The invention relates to a resuscitation device (100) for delivering electric energy to a patient's heart, the device being operable in a defibrillation mode and at least one electric mode different from the defibrillation mode. The device comprises
- at least two electrodes (115) for detecting a rhythm of the patient's heart,
- analysing means for receiving data of a detected rhythm of said at least two electrodes (115) and classifying said data as a shockable or a non-shockable rhythm,
- switching means for switching between the defibrillation mode and the at least one electric mode based on the detected shockable or non-shockable rhythm,
- pulse generator means for generating an electric treatment pulse deliverable to the patient's heart in either the defibrillation mode or the at least one electric mode,
- at least one control unit (101), for defining a treatment pulse in the defibrillation mode or the at least one electric mode,
- a detection unit for detecting chest compression.

The control unit (101) is configured to synchronize the treatment pulse with a detected chest compression.

## Description

The present invention refers to a resuscitation device for delivering electric energy to a patient and a method of operating the device according to the preamble of the independent claim.

Sudden cardiac arrest occurs, when the heart stops being able to pump blood to the vital organs. In this case, performing chest compression is an utmost necessity, eventually quickly followed by the application of an electrical shock to the patient's heart. In the presence of ventricular fibrillation, using a defibrillator may restart the pumping of the heart. In contrast abnormal cardiac rhythm may be treated with lower voltage pacing pulses, which assist the heart's natural pacemakers.

Current Automated External Defibrillators (AED) are computerized devices that help recognizing presence of a shockable rhythm on the patient to whom it is attached. Furthermore, it delivers advice for delivery of Cardiopulmonary Resuscitation by the means of audio and/or visual messages. Basic Life Support (BLS) encompasses the first 3 links in the chain of survival (early access, early CPR, and early defibrillation). Indeed, the AED guides the rescuer to perform CPR at the right moment during the resuscitation process.

The following paragraphs deal with current guidelines and means in order to deliver optimal cardiopulmonary resuscitation (CPR) and helping to improve the quality of the therapy.

### Standard care resuscitation:

The first mission of the non-medical trained rescuer is to recognize the presence of cardiac arrest. Up to 40% of victims are subject to agonal breathing that may be wrongly interpreted as a sign of normal breathing. The guideline is clear in that agonal breathing must not prevent the rescuer from performing CPR.

It is recommended (Perkins et al., Resuscitation, 95, 2015, 81-99.) that the rescuer initiate CPR for presumed cardiac arrest without concerns of harm to patients, even if they may not be in cardiac arrest (strong recommendation, very-low-certainty evidence). The increase in survival chances of starting CPR early outshines the risk of causing minor injuries. The criterion of the BLS to initiate rescue is: if the victim is unresponsive and not breathing normally. The rescuer then calls for help and initiate CPR and makes use of an AED as soon as possible.

The 2015 ERC Guidelines (Perkin et al. 2015) mentions goals for chest compression (CC) on adult patients as follows:
- CC rate of 100 to 120 compressions per minute,
- CC depth of 5 cm (maximum 6 cm),
- CC recoil: release the pressure on the chest after each compression
- Additionally, the amount of time spent in delivery CC should be optimized so that the CC fraction is superior to 60 %.

CPR is the delivery of chest compression and ventilation. A too high number of ventilations is deleterious. CPR is normally delivered in two manners:
a) CPR can be alternated, meaning a series of chest compression and ventilation during 1, 2 or 3 minutes is delivered. For adults, 30 chest compressions are delivered followed by 2 ventilations. For children, 15 chest compressions are delivered, followed by 2 ventilations.
b) CPR can be continuous, meaning that ventilations are provided asynchronously to chest compression, additional hands-off time is avoided. This method is in particular used when the patient receives a tracheal intubation or a laryngeal tube or mask (Advanced life support (ALS) case). The ventilation frequency in that case should be set to 10 per minute.

The purpose of cardiopulmonary resuscitation is to gain time before more advanced medical processes are put in place. Although survival has increased in the last years especially for patients presenting initial shockable rhythm, the survival of patients presenting with non-shockable rhythm remains dismal.

Conventional manual CPR is simple, quick, inexpensive and always available; however, even chest compression provided by trained or experienced rescuers often fails to meet the requirements of guidelines. Mechanical chest compression delivers more consistent and reliable chest compression, eliminates the elements of rescuer fatigue and compression interruption which could potentially overcome the limitations of manual chest compression. Recent clinical studies have demonstrated that the mechanical devices were beneficial to provide continuous chest compression with minimizing no-chest compression intervals during ambulance transportation and interventional coronary catheterization (Bon-nemeier et al., Resuscitation, 82 (2), 2011, 155-59; Wang et al., Resuscitation, 74 (3), 2007, 453-60.) Furthermore, Schiller has previously demonstrated that mechanical chest compression with Easy Pulse from Schiller improved hemodynamic efficacy and the success of CPR, and therefore achieved better post-resuscitation myocardial and neurological recovery (Chen et al., Critical Care Medicine, 40(11), 2012, 3007-12).

CPR quality can be improved by several means (paragraph "Feedback on compression technique" in Perkins et al. 2015). Guidelines emphasize that first a broad system of care should include CPR improvement initiative. In other terms, the highest priority to improve CPR is to deliver feedback advices to the rescuer teams after interventions, during debriefing sessions. A peculiar attention will also be given to CC fraction (Couper et al., Intensive Care Medicine, 39 (9), 2013, 1513-23.). This measurement can be performed using transthoracic impedance or sternal sensors. When such process is in place, one can improve CPR in real time during the intervention using either thoracic impedance-based measurements or sternal sensors.

### Electric stimulation means: Transthoracic pacing

Work relating to transthoracic stimulation (pacing) during cardiac arrests is quite rare and rather old. The survival of cardiac arrest patients treated with transthoracic stimulation is very low. This can be explained by several factors. First of all, the available studies present rather poorly the critical times known to date to be crucial during cardiac arrests, or the times for the implementation of stimulation are very long (> 15 minutes). Most clinical studies involve several types of patients requiring treatment with transthoracic stimulation: bradyasystolias after electric shocks on ventricular fibrillation, unstable bradycardias, cardiac arrests by asystole. According to studies, "complete block" rhythms may or may not be part of asystoles. Thus, the dispersion of patients in small cohorts, implies a decrease in the relevance of the results. Most of the devices used are external pacemakers and do not include a defibrillation function. Thus, it is necessary for the rescuer to make a therapeutic choice based on an analysis of monitoring data. This implies a potential loss of time due to the installation of the monitoring system, the clinical evaluation of the patient, the placement of the external pacemaker before initiating treatment with it.

Finally, a review of the literature on cardiac arrests due to bradyasystole (Sherbino et al., Resuscitation 70(2), 2006, 193-200.) showed that this subject was only dealt with by studies with a low level of evidence.

In a comparative study, Cummins et al (Cummins et al., The New England Journal of Medicine 328 (19), 1377-82) reported that primary asystole and asystole present following VF shock appeared to have an identical and low survival profile. The group of patients randomized to receive transthoracic stimulation consisted of 278 patients of whom only 112 had received the electrical treatment. The author has not disclosed any specific survival information for these 112 asystolic patients treated with transthoracic stimulation.

In all studies, the duration of pacing pulses is either 20 ms or 40 ms. The amplitude is set by the user at different values (0 to 200 mA) depending on the patient's response to the stimulation.

### Full electric CPR

Work relating to electrical CPR during cardiac arrests is very rare. The only study describing such process was published by Wang et al (H. Wang et al., Critical Care Medicine 36 (Suppl): S458-66, 2008). The purpose of the medical device is to perform chest compressions to a patient without mechanical action on the chest of any bystander. It is an animal study and it discloses two types of stimulation impulses called either skeletal-based or cardiac-based presenting pulse trains of variable duration, each internal pulse being respectively 0.15 ms or 7.5 ms wide (pulse width). The amplitude of impulses is around 2 A. The study describes the effect of several parameters (pulse width, pulse period, train width cardiac output). The study shows that some settings of electrical pulses are leading to similar coronary perfusion pressure (CPP) as manual chest compression (MCC). For instance pulse trains as follows:
- pulse width = 0.15 ms, pulse period = 15 ms, train width = 200 ms, train rate = 120/min
- pulse width = 7.5 ms, pulse period = 30 ms, train width = 200 ms, train rate = 120/min
allow to reach respectively a CPP of 19.3 +/-9.0 mmHg and 22.3 +/-11.6 mmHg whereas MCC in the same conditions of test is reaching 22.9 +/-9.4 mmHg. However, the feasibility of the disclosed process is described for long term electrical stimulation.

It is at least one objective of the present invention to overcome the drawbacks of the prior art. In particular it is one objective to provide a resuscitation device that combines non-invasive stimulation with a defibrillator and helps to further optimize CPR.

The at least one objective is solved by the subject-matter of the independent claims. Preferred embodiments are described with regard to the dependent claims.

A first aspect of the invention refers to a resuscitation device for delivering electric energy to a patient. The device is operable in a defibrillation mode and at least one electric mode different from the defibrillation mode. The resuscitation device comprises
- at least two electrodes for detecting a rhythm of the patient's heart,
- analysing means for receiving data of a detected rhythm of said at least two electrodes and classifying said data as a shockable or a non-shockable rhythm,
- switching means for switching between the defibrillation mode and the at least one electric mode based on the detected shockable or non-shockable rhythm,
- pulse generator means for generating an electric treatment pulse in either the defibrillation mode or the at least one electric mode,
- at least one control unit, for defining a treatment pulse in the defibrillation mode or the at least one electric mode,
- a detection unit for detecting chest compression, characterized in that the control unit is configured to synchronize the treatment pulse with a detected chest compression.

By "synchronize" it is meant that the treatment pulse is delivered at the appropriate moment during chest compression. For example, based on the processing of the detected rhythm, the device can detect the start of chest compression. A certain delay is waited before delivering the treatment pulse at the appropriate moment of the compression cycle.

The electric treatment pulse can be delivered to the patient's heart or to surrounding muscles. Defibrillation is always aiming at being delivered to the patient's heart.

The term "mode" refers to a time frame which starts with the detection of the rhythm and last until the next detection of a rhythm. For example, if a shockable rhythm is detected, the device switches into the defibrillation mode and remains in this mode until the next detection. Thus, the device may remain in the defibrillation mode beyond applying a defibrillation pulse as treatment pulse.

The chest compression can be manual chest compression, e.g. performed by a rescuer or automated chest compression. Chest compression with the help of a mechanical device is also possible.

Thus, the device has the advantage that based on the detected rhythm the right treatment pulse is delivered. Even if a non-shockable rhythm is detected, a treatment pulse can be delivered without interruption of chest compression, providing a patient optimized treatment protocol.

Preferably, the device comprises one control unit, shared between the two operation modes. The device may also comprise two control units, one for each mode.

Advantageously, the at least one electric mode is a pacing mode. Typically, the pacing pulse duration can be between 20 - 40 ms, the amplitude can vary between 5 mA to 200 mA.

Pacing during chest compression is believed to represent a very effective treatment for several types of non-shockable rhythms.

Pacing can also be possible in case of defibrillation, where defibrillation is e.g. followed by CPR including continuous pacing synchronized with chest compression.

However, it is also possible to use an electric mode different from classic pacing modes. In this case, the pulse duration may be the same, but amplitude can be between 1-2 A. Such amplitudes are not typical for cardiac pacing. The waveform of the electric pulse can be rectangular, squared, chopped, exponential or, depending on the capacitor, bi-phasic.

The analysing means can be formed by an ECG detection unit and the at least two electrodes can be electrical coupled to the ECG detection unit.

An ECG is a straightforward way of identifying shockable and non-shockable rhythm and is easy to implement in a resuscitation device.

Preferably, the pulse generator means comprises at least one capacitor, preferably a high-voltage capacitor. The capacitor can be shared between the modes, or alternatively, a capacitor for each mode can be present.

The device may contain an H-bridge comprising four switches A, B, C and D. The treatment pulse can be applicable to a load outside the apparatus through the H-bridge.

An input of each switch A and B can be linked to one side of the at least one capacitor and an input of each switch C and D can be linked to the other side of the at least one capacitor.

The term "linked" means that the switch can be in an open state, interrupting the electric circuit or in close state closing the electric circuit.

Advantageously, an output of each switch A and C is coupled to one electrode of the at least two electrodes and an output of each switch B and C is coupled to the other electrode of the at least two electrodes.

Preferably, in the defibrillation mode, the capacitor is connected to the inputs of switch A and B and the pair of switches A + D and B + C is being used for the first and second phase of a biphasic defibrillation pulse.

The device can comprise a pacer current regulation circuit and, in the pacing mode, the at least one capacitor can be connected to the input of switch B and an output of the H-Bridge can be electrically connected to the pacer current regulation circuit for regulating the energy in the pacing mode.

The current regulation circuit allows a constant current supply, making the device safer in the pacing mode.

The connection of the capacitor to the switches of the H-bridge can vary and may depend on the type of electric treatment pulse delivered, such as alternate stimulation pulses or chopped pulses.

The detection unit for detecting chest compression can comprise means for detecting chest compression, preferably selected from the group consisting of: a gyroscope, an accelerometer, an.impedance meter, piezoelectric-based means, strain-gauge means or a camera.

Preferably, each electrode is disposed within at least one patch, preferably an adhesive patch. Current can flow between the two patches applied on the chest of the patient. Patch refers to the skin applied essentially flat part for fixing the electrodes to the skin. Current discharge may occur via the patches. Each electrode is operable in the defibrillation mode or the pacing mode, or alternatively, the patch comprises a separate electrode for each mode.

The at least one patch can comprise the means for detecting chest compression. The patch may also be configured to detect impedance variations of the patient, resulting from chest compression.

The patches can be placed on different positions of the patient's body according to the resuscitation guidelines; sterno-apical, anterio-posterior, bi-axillary.

Advantageously, the device is a portable defibrillator device. The portable device may provide a common housing for the above described components. Such a device would be compact and could easily be available in a non-medical environment such as shopping streets, stations or office buildings. Preferably, in such a portable device, the circuits are shared between the components.

The device can be connected or connectable to a chest compression unit, in particular a chest compression unit comprising an automatic actuation. Even more preferred, the chest compression unit and the resuscitation device can be in electrical communication such that an automatic chest compression synchronizes with a treatment pulse or vice versa. The chest compression unit can also comprise manual chest compression means for assisting a rescuer in performing chest compression.

A mechanical chest compression device could be Schiller Easy Pulse, a manual chest compression device could be CardioPump^{®}.

A second aspect of the invention refers to a resuscitation system comprising a resuscitation device as previously described and a chest compression unit, preferably with an automatic actuation. As described above, the resuscitation device and the chest compression unit are in electrical communication. Preferably, the control unit may control the function of the resuscitation device as well as the chest compression unit. It is also possible that the chest compression unit comprises a separate control unit allowing signal exchange with the resuscitation device and preferable with the control unit of the resuscitation device to allow synchronization of chest compression and treatment pulse as previously described.

Such an arrangement would allow an improved clinical quality of chest compression. An almost fully automatic resuscitation system without any decisive action on the part of the rescuer, apart from connecting the device to the patient, may reduce time loss during CPR.

The chest compression unit may also comprise mechanical, chest compression means for assisting a rescuer in performing the chest compression correct and sufficiently strong. Mechanical chest compression allows to minimize the time without chest compression once in place. It delivers consistent chest compression in time and keeps quality of chest compression during transportation.

A third aspect of the invention refers to a method of operating a resuscitation device as previously described. The method comprising the steps of
- Detecting a rhythm of a patient's heart,
- Classifying said rhythm for a shockable or a non-shockable rhythm,
- Switching into the defibrillation mode if a shockable rhythm is detected or switching into an electric mode different from the defibrillation mode if a non-shockable rhythm is detected,
- Synchronizing a delivery of a first electric treatment pulse in the electric mode for non-shockable rhythm with chest compression performed on the patient.

Chest compression can be performed manually, with or without the help of a cardio pump. Chest compression can also be performed mechanically or fully automated as described above.

The method may further comprise the step of delivering a second electric treatment pulse in the defibrillation mode, preferably, the electric treatment pulse is synchronized with chest compression.

Further, the delivery of an electric pulse, in particular pacing, is not limited to classifying a rhythm as non-shockable. Pacing may also be performed after a shockable rhythm had been detected and defibrillation was performed. Defibrillation can be followed by continuous pacing.

In general and as described further above, the respective mode is not limited to applying the electric treatment pulse but can include further CPR or only chest compression before a new rhythm is detected.

The method provides an optimized CPR treatment, taking the present heart rhythm and health state of the patient into account.

The invention is described in more details with regard to the following figures. The figures are only preferred embodiments and are not to be understood as limiting. It shows:
- Figure 1: A block diagram of the electric circuit of the resuscitation device according to the invention.
- Figure 2: Shows a graphic representation of the synchronization of chest compression and electric stimulation with a treatment pulse.

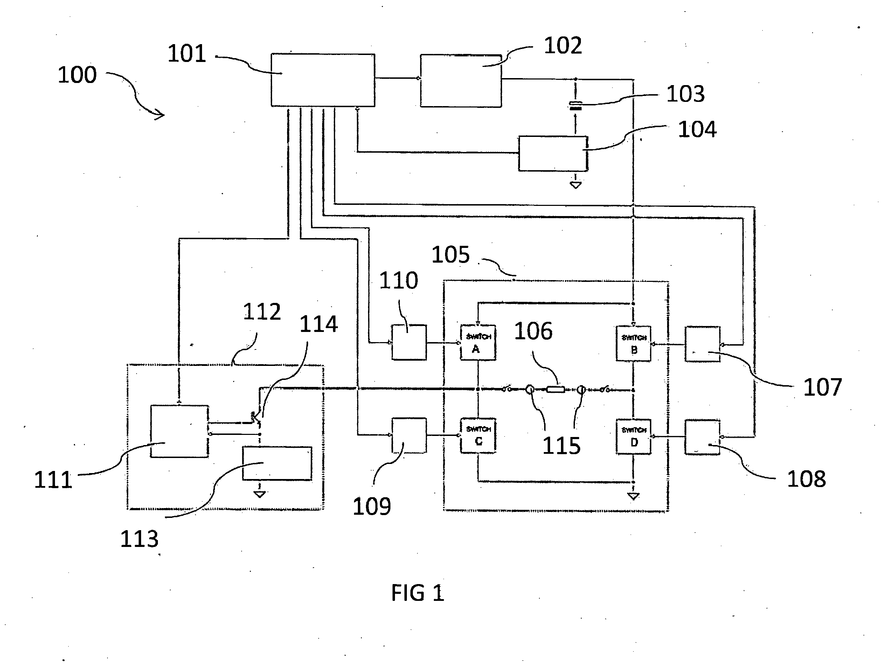

Figure 1 shows a block diagram of a resuscitation device 100 that incorporates a defibrillator and a pacing control unit 101 in one device. The device comprises common components for working in either a defibrillation mode or a pacing mode, including a high voltage (HV) capacitor 103, a capacitor charging unit 102 and a H-bridge 105. The capacitor 103 is connected to an input of the H-Bridge 105. The H-bridge circuit 105 has four switches A-D and can be coupled to the patient 106 via two electrodes 115. The electrodes 115 are applied to the chest of the patient 106. The H-bridge 105 delivers electrical current from the capacitor 103 to the patient 106. The H-bridge 105 has two modes of operation, one for defibrillation and one for pacing. A switch control circuit 107-110 for each switch A-D controls the switches for the individual modes. Each switch control circuit 107-110 is regulated by the defibrillator and pacing control unit 101, which controls the polarity of the electrical current delivered to the patient 106 in the defibrillation mode (biphasic defibrillation shock delivery). A pacer current regulation system 112 is arranged in serial connection with the center tab of the H-bridge 105.

In the defibrillation mode, the HV capacitor 103 is connected to the input of switch A and B of the H-bridge 105. The H-bridge 105 is coupled with the patient 106 and delivers a defibrillation pulse of electrical energy to the patient 106 via a pair of electrodes 115. A defibrillator pulse basically known in the art is delivered by either turning on switch A and D or B and C. In particular, sequentially switching either switches A and D or B and C creates a biphasic pulse for defibrillation. The output of switches C and D is coupled to a GND potential.

In the pacing mode, the H-bridge 105 delivers electrical current from the capacitor 103 to the patient 106 by only switching on switch B. In the pacing mode, the output of the H-bridge 105 is electrically connected to a pacer current regulation circuit 112 via the node between switch A and C. The pacer current regulation circuit 112 regulates the energy during the pacing mode and is controlled by the defibrillator/pacer control unit 101 via a DAC or PWM output signal. A transistor 114 in the pacer current regulating circuit 112 controls the incoming current from switch B. The pacer current is measured via the Pacer current measurement resistor 113 connected to the fix GND potential. Depending on the measured current, the current source control circuit 111 receives a respective signal and delivers a feedback to the transistor 114 to regulate the current. The output of the transistor 114 is connected to the fix GND potential (see block diagram). This connection can be suppressed. Pacing is controlled by closing switch B 200 µs before the Current Source control circuit 111 is activated. Closing is controlled by an external trigger, e.g. chest compression. The current Source control circuit 111 then generates the constant 20ms Pacer pulse duration.

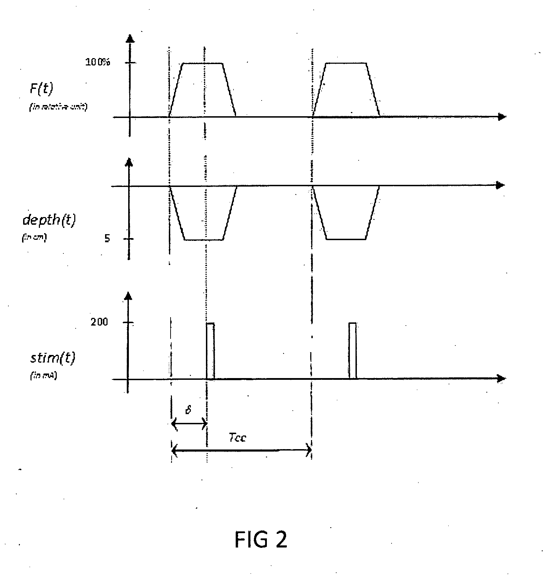

Figure 2 is a graphic representation of the synchronization of the chest compression with the pacing stimuli. F(t) represents the application force of the chest compression in relative units, depth (t) is the motion distance of the chest related to the compression in cm. Stim (t) is the stimulation current, such as a pacing current, delivered at a delay δ after the onset of the chest compression. T_{cc} is the period of chest compression. The delay d can be found between 0 and T_{cc}. The delay can be fixed and stable in time all along the duration of the intervention on the patient. The delay δ can be variable in time during the intervention on the patient, depending on other physiological information.

A stimulation current that is used in standard non-invasive transthoracic pacing has a maximum current of usually 200 mA for a duration of 20 or 40 ms depending on the device. in the present invention alternate currents can also be used up to 5 A. The stimulation pulse, which is also referred to as treatment pulse, can be composed of several smaller impulses composing a pulse train delivered regularly.

## Claims

1. Resuscitation device (100) for delivering electric energy to a patient, the device being operable in a defibrillation mode and at least one electric mode different from the defibrillation mode, wherein the device comprises
- at least two electrodes (115) for detecting a rhythm of the patient's heart,
- analysing means for receiving data of a detected rhythm of said at least two electrodes (115) and classifying said data as a shockable or a non-shockable rhythm,
- switching means for switching between the defibrillation mode and the at least one electric mode based on the detected shockable or non-shockable rhythm,
- pulse generator means for generating an electric treatment pulse in either the defibrillation mode or the at least one electric mode,
- at least one control,unit (101), for defining a treatment pulse in the defibrillation mode or the at least one electric mode,
- a detection unit for detecting chest compression, **characterized in that** the control unit (101) is configured to synchronize the treatment pulse with a detected chest compression.

2. Resuscitation device (100) according to one of the previous claims, wherein the at least one electric mode is a pacing mode.

3. Resuscitation device (100) according to one of the previous claims, wherein the analysing means are formed by an ECG detection unit and the at least two electrodes (115) are electrical coupled to the ECG detection unit.

4. Resuscitation device (100) according to one of the previous claims, wherein said pulse generator means comprises at least one capacitor (103), preferably a high-voltage capacitor.

5. Resuscitation device (100) according to one of the previous claims wherein the device contains an H-bridge (105) comprising four switches A, B, C and D and wherein the treatment pulse is applicable to a load outside the apparatus through the H-bridge (105).

6. Resuscitation device (101) according to claim 5, wherein an input of each switch A and B is linked to one side of the at least one capacitor (103) and wherein an input of each switch C and D is linked to the other side of the at least one capacitor (103).

7. Resuscitation device (100) according to claim 5 or 6, wherein an output of each switch A and C is coupled to one electrode of the at least two electrodes (115) and wherein an output of each switch B and C is coupled to the other electrode of the at least two electrodes (115).

8. Resuscitation device (100) according to one of claims 5 to 7, wherein, in the defibrillation mode, the capacitor (103) is connected to the inputs of switch A and B and the pair of switches A + D and B + C being used for the first and second phase of a biphasic defibrillation pulse.

9. Resuscitation device (100) according to one of claim 5 to 8, wherein, the device comprises a pacer current regulation circuit (112) and, in the pacing mode, the at least one capacitor (103) is connected to the input of switch B and an output of the H-Bridge (105) is electrically connected to the pacer current regulation circuit (112) for regulating the energy in the pacing mode.

10. Resuscitation device (100) according to one of the previous claims, wherein the detection unit for detecting chest compression comprises means for detecting chest compression, preferably selected from the group consisting of: a gyroscope, an accelerometer, an impedance meter, piezoelectric-based means, strain-gauge means or a camera.

11. Resuscitation device (100) according to one of the previous claims, wherein the device is a portable defibrillator device.

12. Resuscitation device (100) according to one of the previous claims, wherein the device is connected or connectable to a chest compression unit, in particular a chest compression unit comprising an automatic actuation.

13. Resuscitation system comprising a resuscitation device (100) according to one of claims 1 to 12 and a chest compression unit, preferably with an automatic actuation.

14. Method of operating a resuscitation device (100) according to one of claims 1 to 12, the method comprising the steps of
- Detecting a rhythm of a patient's heart,
- Classifying said rhythm for a shockable or a non-shockable rhythm,
- Switching into the defibrillation mode if a shockable rhythm is detected or switching into an electric mode different from the defibrillation mode if a non-shockable rhythm is detected,
- Synchronizing a delivery of a first electric treatment pulse in the electric mode for non-shockable rhythm with chest compression performed on the patient.

15. Method according to claim 14, further comprising the step of delivering a second electric treatment pulse in the defibrillation mode, preferably, the electric treatment pulse is synchronized with chest compression.
